# EUROPEAN PATENT SPECIFICATION

(11) **EP 2 496 710 B1**
(45) Date of publication and mention of the grant of the patent: **02.07.2014**
(21) Application number: 10771486.7
(22) Date of filing: 02.11.2010
(51) Int. Cl.: C12Q 1/68

(54) **DETERMINATION OF 17Q GAIN IN NEUROBLASTOMA PATIENTS BY ANALYSIS OF CIRCULATING DNA**
BESTIMMUNG DER 17Q-ZUNAHME BEI NEUROBLASTOM-PATIENTEN MITTELS ANALYSE DER ZIRKULIERENDEN DNA
DÉTERMINATION DU GAIN 17Q DANS LE NEUROBLASTOME DE PATIENTS PAR L'ANALYSE DE L'ADN CIRCULANT

(30) Priority: 02.11.2009 EP 09306045
(43) Date of publication of application: 12.09.2012
(73) Proprietor: Centre Léon Bérard, 69008 Lyon (FR)
(72) Inventor: PUISIEUX, Alain, F-38300 Ruy Montceau (FR); COMBARET, Valérie, F-69002 Lyon (FR)
(74) Representative: Regimbeau
(86) International application number: PCT/EP2010/066627
(87) International publication number: WO 2011/051495

(56) References cited:
- WO-A2-03/014319
- WO-A2-2006/050732
- MOROWITZ MICHAEL ET AL: "Detection of single-copy chromosome 17q gain in human neuroblastomas using real-time quantitative polymerase chain reaction.", MODERN PATHOLOGY, vol. 16, no. 12, December 2003 (2003-12), pages 1248-1256, XP002566134, ISSN: 0893-3952
- GOTOH T ET AL: "In reply [8]", JOURNAL OF CLINICAL ONCOLOGY 2005 US, vol. 23, no. 34, 2005, page 8920, XP002566135, ISSN: 0732-183X
- TAJIRI ET AL: "Biological diagnosis for neuroblastoma using the combination of highly sensitive analysis of prognostic factors", JOURNAL OF PEDIATRIC SURGERY, W. B. SAUNDERS COMPANY, US, vol. 41, no. 3, 1 March 2006 (2006-03-01), pages 560-566, XP005312152, ISSN: 0022-3468
- GOTOH TAKAHIRO ET AL: "Prediction of MYCN amplification in neuroblastoma using serum DNA and real-time quantitative polymerase chain reaction.", JOURNAL OF CLINICAL ONCOLOGY : OFFICIAL JOURNAL OF THE AMERICAN SOCIETY OF CLINICAL ONCOLOGY 1 AUG 2005, vol. 23, no. 22, 1 August 2005 (2005-08-01), pages 5205-5210, XP002566136, ISSN: 0732-183X cited in the application
- COMBARET VALERIE ET AL: "Influence of neuroblastoma stage on serum-based detection of MYCN amplification.", PEDIATRIC BLOOD & CANCER SEP 2009, vol. 53, no. 3, September 2009 (2009-09), pages 329-331, XP002566137, ISSN: 1545-5017 cited in the application

### SEQUENCE LISTING

<110> CENTRE LEON BERARD
<120> Determination of 17q gain in neuroblastoma patients by analysis of circulating DNA
<130> 357245D28064 KH
<150> EP09306045.7
   <151> 2009-11-02
<160> 18
<170> PatentIn version 3.3
<210> 1
   <211> 18
   <212> DNA
   <213> Artificial
<220>
   <223> primer
<400> 1
   agggctcctg gccattct 18
<210> 2
   <211> 21
   <212> DNA
   <213> Artificial
<220>
   <223> primer
<400> 2
   aaccacccca acacaccatc t 21
<210> 3
   <211> 27
   <212> DNA
   <213> Artificial
<220>
   <223> probe
<400> 3
   aggtgctgct ccaggtaaca gttccca 27
<210> 4
   <211> 20
   <212> DNA
   <213> Artificial
<220>
   <223> primer
<400> 4
   tgtccttcct ggagcgatct 20
<210> 5
   <211> 22
   <212> DNA
   <213> Artificial
<220>
   <223> primer
<400> 5
   caaacccctg gtttagcact tc 22
<210> 6
   <211> 20
   <212> DNA
   <213> Artificial
<220>
   <223> probe
<400> 6
   cagcccccgg ctccgctaga 20
<210> 7
   <211> 16
   <212> DNA
   <213> Artificial
<220>
   <223> primer
<400> 7
   gggctgccac gtccac 16
<210> 8
   <211> 18
   <212> DNA
   <213> Artificial
<220>
   <223> primer
<400> 8
   gtcgtcatct ggctccca 18
<210> 9
   <211> 26
   <212> DNA
   <213> Artificial
<220>
   <223> probe
<400> 9
   ttcatccact gccccactga gaacga 26
<210> 10
   <211> 18
   <212> DNA
   <213> Artificial
<220>
   <223> primer
<400> 10
   cggtccccca cctctctt 18
<210> 11
   <211> 22
   <212> DNA
   <213> Artificial
<220>
   <223> primer
<400> 11
   cggtttagcc accaactttc tc 22
<210> 12
   <211> 16
   <212> DNA
   <213> Artificial
<220>
   <223> probe
<400> 12
   cctcagactg cgctgc 16
<210> 13
   <211> 20
   <212> DNA
   <213> Artificial
<220>
   <223> primer
<400> 13
   gtgctctcca attctcgcct 20
<210> 14
   <211> 19
   <212> DNA
   <213> Artificial
<220>
   <223> primer
<400> 14
   gatggcctag aggagggct 19
<210> 15
   <211> 26
   <212> DNA
   <213> Artificial
<220>
   <223> probe
<400> 15
   cactaaagtt ccttccaccc tctcct 26
<210> 16
   <211> 20
   <212> DNA
   <213> Artificial
<220>
   <223> primer
<400> 16
   tgggcagaca catcgtagca 20
<210> 17
   <211> 21
   <212> DNA
   <213> Artificial
<220>
   <223> primer
<400> 17
   caccttcact cccacctcaa c 21
<210> 18
   <211> 22
   <212> DNA
   <213> Artificial
<220>
   <223> probe
<400> 18
   tgttgcccga gattgacccg gt 22

## Description

Neuroblastoma (NB), the most frequent extracranial solid tumor of childhood, is characterized by wide clinical variability, with possible cellular maturation or spontaneous tumor regression on the one hand, or aggressive clinical behavior with rapid progression despite intensive therapeutic approaches on the other hand. The International Neuroblastoma Staging System currently stratifies patients into very low, low-, intermediate-, or high-risk categories based upon well-defined prognostic factors. These include patient age at diagnosis, disease stage, histopathological diagnosis, and MYCN oncogene status.

However, the MYCN amplification is not the unique genomic alteration to identify aggressive tumors. Recently, different retrospective studies using comparative genomic hybridization (CGH) or array CGH (aCGH) have shown that the presence of segmental alterations is the strongest predictor of relapse. (1-4). The gain of chromosome arm 17q has been shown to be the most frequent chromosome alteration in neuroblastoma primary tumors, occurring in 50%-70% of cases (5-8). It is associated with a poor prognosis and constitutes an independent indicator of adverse outcome, with a predictive value stronger than that of all other clinical, histopathologic, and genetic markers (5, 9).

In several types of neoplasias, molecular techniques, such as polymerase chain reaction (PCR), have detected small amounts of free DNA in the serum and plasma of patients with melanoma, breast, head and neck or lung cancer suggesting that circulating DNA is a valuable target for cancer marker detection (10 - 17). Recently, we and others demonstrated that high levels of circulating MYCN DNA sequences, as assessed by real-time quantitative PCR, were present in the peripheral blood of patients with MYCN-amplified (MNA) neuroblastoma (18-21).

Whereas the MYCN alteration is a multicopy amplification, the gain of genetic material from chromosome arm 17q is an unbalanced translocation with a variety of partner chromosomes. The segment on the partner chromosome distal to the breakpoint is lost, and a segment of 17q translocates to that site. The translocated segment is in effect an extra copy, because the cell also contains two or more normal chromosomes 17. These translocations therefore result in an unbalanced gain of the distal segment of 17q (5). Contrary to MYCN alterations, detection of 17q gain in circulating DNA has never been performed because it was anticipated that this technique would not exhibit sufficient sensitivity for effective prognosis of neuroblastoma. Further, a clear-cut correlation between 17q gain and prognosis of neuroblastoma had never been established.

Morowitz et al (Modern Pathology, 1248-1256, 2003) describes detection of 17q gain using RT quantitative PCR on tumor samples. This document further teaches that there the tumors have to be studied by FISH for unequivocal determination of 17q allelic status (see page 1255 of Morowitz et al.).

Gotoh et al. (J.Clinical Oncology, 8920, 2005) discusses a serum *MYCN PCR* assay as a diagnostic test. Detection of 17q gain in serum is neither described nor suggested. Whereas the MYCN alteration is a multicopy amplification, the gain of genetic material from chromosome arm 17q is an unbalanced translocation with a variety of partner chromosomes.

Tajiri et al. (J. Pediatric Surgery, 560-566, 2006) describes the use of a combination of biological markers for establishing a prognosis of neuroblastoma. The prognostic value of 17q gain detected in the serum in infants and toddlers of less than 18 months of age is not described.

Gotoh et al. (J. Clinical Oncology, 5205-5210, 2005) describes prediction *of MYCN* amplification in neuroblastoma using serum DNA and RT quantitative PCR. Combaret et al. (Pediatr Blood Cancer, 53:329-331, 2009) discusses the influence of neuroblastoma stage on serum-based detection of *MYCN* amplification. Detection of 17q gain in serum is neither described nor suggested in these documents. Whereas the MYCN alteration is a multicopy amplification, the gain of genetic material from chromosome arm 17q is an unbalanced translocation with a variety of partner chromosomes.

Surprisingly, the present data shows that 17q gain is detected in the peripheral blood of neuroblastoma patients. The sensitivity which was obtained is sufficient to provide valuable information to the clinician. Unexpectedly, the specificity of 17q gain determination was highly relevant in infants and toddlers under 18 months of age compared to infants and toddlers over 18 months of age. These results show that 17q gain determination in circulating DNA is feasible demonstrating that this non invasive assay could be useful for infants and toddlers with neuroblastoma for which the *"wait and see"* strategy is often recommended. It could help to define the prognosis and therapy stratification especially for patients without MYCN amplification neuroblastoma. This assay is complementary to assays developed previously to detect circulating MYCN DNA sequences.

### SUMMARY OF THE INVENTION

The present invention relates to a method for diagnosis of neuroblastoma comprising determining whether there is a gain of genetic material from chromosome segment 17q21-qter in a sample of circulating nucleic acids from a human subject.

Preferably, the methods of the present invention are *in vitro* methods.

Preferably, the sample of circulating nucleic acids is a sample of circulating DNA.

Preferably, the sample of circulating nucleic acids is from a human subject under 18 months of age.

Preferably, the sample of circulating nucleic acids is negative for MYCN gene amplification.

Preferably, the gain of genetic material from chromosome segment 17q21-qter is determined by quantification of the MPO gene located on 17q23.1 in comparison with a reference gene.

Preferably, the gain of genetic material from chromosome arm segment 17q21-qter is determined by quantification of the SURVIVIN gene located on 17q25 in comparison with a reference gene.

Preferably, the reference gene is selected from a gene on chromosome 17. Preferably, the reference gene is p53 located on 17p13.1.

In preferred embodiments, a gain of genetic material from chromosome segment 17q21-qter is determined by quantitative amplification methods.

In one embodiment, a gain of genetic material from chromosome arm segment 17q21-qter is determined by quantitative PCR using primers of SEQ ID Nos. 1-2 and probe of SEQ ID no. 3.

In another embodiment, a gain of genetic material from chromosome arm segment 17q21-qter is determined by quantitative PCR using primers of SEQ ID Nos. 7-8 and probe of SEQ ID no. 9.

In some embodiments, a gain of genetic material from chromosome arm segment 17q21-qter is associated with a poor prognosis.

In other embodiments, a gain of genetic material from chromosome arm segment 17q21-qter is associated with reduced Progression Free Survival (PFS).

In other embodiments, a gain of genetic material from chromosome arm segment 17q21-qter is associated with reduced Overall Survival (OS).

### DESCRIPTION OF THE SEQUENCE LISTING

| | |
|---|---|
| SEQ ID No. 1 | MPO forward primer |
| SEQ ID No. 2 | MPO reverse primer |
| SEQ ID No. 3 | MPO probe |
| SEQ ID No. 4 | p53 forward primer |
| SEQ ID No. 5 | p53 reverse primer |
| SEQ ID No. 6 | p53 probe |
| SEQ ID No. 7 | Survivin forward primer |
| SEQ ID No. 8 | Survivin reverse primer |
| SEQ ID No. 9 | Survivin probe |
| SEQ ID No. 10 | MYCN forward primer 1 |
| SEQ ID No. 11 | MYCN reverse primer 1 |
| SEQ ID No. 12 | MYCN probe 1 |
| SEQ ID No. 13 | MYCN forward primer 2 |
| SEQ ID No. 14 | MYCN reverse primer 2 |
| SEQ ID No. 15 | MYCN probe 2 |
| SEQ ID No. 16 | NAGK forward primer |
| SEQ ID No. 17 | NAGK reverse primer |
| SEQ ID No. 18 | NAGK probe |

### DETAILED DESCRIPTION OF THE INVENTION

Neuroblastoma is the most common extracranial solid cancer in childhood and the most common cancer in infancy. Neuroblastoma cases often occur in children younger than two years old and it is one of the few human malignancies known to demonstrate spontaneous regression. Diagnostic and prognostic tools for neuroblastoma are therefore of outmost importance for clinicians to decide on the appropriate therapeutic approach. Gain of 17q has been described as a prognostic marker for neuroblastoma. In particular, gain of 17q in neuroblastoma is associated with a poor prognosis for progression free survival and/or with a poor prognosis for overall survival.

The present invention shows that gain of 17q is surprisingly detected with a good sensitivity and specificity in circulating nucleic acids, especially in infants and toddlers less than 18 months of age.

In a first embodiment, the present invention is related to a method for diagnosis of neuroblastoma comprising determining whether there is a gain of genetic material from chromosome arm segment 17q21-qter in a circulating nucleic acid sample from a human subject.

Preferably, the methods of the present invention are *in vitro* methods. The methods of the present invention are carried out on a biological sample previously taken from a patient. Typically, the patient has been diagnosed with neuroblastoma or is suspected of suffering from neuroblastoma.

The term "diagnosis" refers both to diagnosis and prognosis of neuroblastoma.

In the methods of the present invention, gain of 17q is detected in circulating nucleic acids. The terms "circulating nucleic acids" refer both to free RNA and DNA circulating in the blood. Preferably, free circulating nucleic acids are obtained from a blood sample, a serum sample, a plasma sample or any other appropriate biological sample containing free circulating nucleic acids. Preferably, the sample taken from the patient is a plasma sample or a serum sample. Preferably, gain of 17q is detected in circulating DNA.

Gain of 17q is a chromosome alteration in neuroblastoma tumors. The gain of genetic material from chromosome arm 17q is an unbalanced translocation with a variety of partner chromosomes. The segment on the partner chromosome distal to the breakpoint is generally lost, and a segment of 17q translocates to that site. The translocated segment is in effect an extra copy, because the cell also contains two or more normal chromosomes 17. These translocations therefore result in an unbalanced gain of the distal segment of 17q (5). The terms "gain of 17q" refer to gain of genetic material from chromosome arm segment 17q21-qter.

The methods of the present invention, performed on circulating DNA, surprisingly exhibit a good sensitivity and a good specificity especially in patients under 2 years of age, in patients under 12 months of age and more preferably in patients under 18 months of age.

Sensitivity and specificity are statistical measures of the performance of a binary classification test.

As used herein, "sensitivity" measures the proportion of patients having a gain of 17q as detected on the tumor, which are correctly identified as such by a test determining gain of 17q on circulating DNA.

As used herein, "specificity" measures the proportion of patients lacking a gain of 17q as detected on the tumor, which are correctly identified as such by a test determining gain of 17q on circulating DNA.

In the methods of the present invention, the circulating DNA sample is preferably from a human subject under 2 years of age, preferably from a subject under 12 months of age and more preferably from a human subject under 18 months of age.

MYCN oncogene amplification has been described as a genetic marker for neuroblastoma prognosis. MYCN amplified neuroblastomas are associated with a poor prognosis and progressing tumors. MYCN oncogene status is used, with other factors, to classify patients into very low, low, intermediate or high risk categories.

For some patients, in particular for infants and toddlers, a wait and see strategy may be applied. In these cases, a tumor sample from the patient is not available for further characterization of the tumor and for analysis of the genetic alterations in the tumor cells. A new method has been described for analysis of MYCN gene status in circulating DNA (18). However, some neuroblastoma cases without MYCN amplification may turn out to have a poor prognosis. Detection of 17q gain in circulating nucleic acids is therefore a useful tool to detect at least some cases of non MYCN amplified neuroblastoma with poor prognosis. In preferred embodiments of the present invention, patients or biological samples of circulating nucleic acids from patients diagnosed with neuroblastoma or suspected of suffering from neuroblastoma, are first tested for MYCN amplification and, if no MYCN amplification is detected, are further tested for gain of 17q.

In preferred embodiments, the circulating nucleic acid sample is negative for MYCN amplification. The methods of the present invention are preferably carried out on circulating nucleic acids from patients with non MYCN amplified neuroblastoma. Preferably, in the methods of the present invention, there is no amplification of the MYCN gene in the circulating nucleic acids (i.e. in the circulating DNA).

MYCN gene amplification may be detected according to any known method. MYCN amplification may be detected on a tumor sample or on circulating DNA. Preferably, MYCN amplification is detected on circulating DNA as described by Combaret *et al.* (18). The GENBANK accession number for the MYCN oncogene is NG_007457.

MYCN gene amplification is detected by an appropriate method such as quantitative amplification methods.

In one embodiment, MYCN amplification is detected by quantitative PCR using the forward and reverse primers of SEQ ID Nos. 10-11 and the quantitative PCR (TaqMan™) probe of SEQ ID No. 12. MYCN amplification is detected by Taqman™ PCR using forward primer 5'-CGGTCCCCCACCTCTCTT-3', reverse primer 5'-CGGTTTAGCCACCAACTTTCTC-3' and probe 5'-FAM-CCTCAGACTGCGCTGC-TAMRA-3' as described by Combaret *et al* (18).

In another preferred embodiment, MYCN amplification is detected by quantitative PCR using the forward and reverse primers of SEQ ID Nos. 13-14 and the quantitative PCR (TaqMan™) probe of SEQ ID No. 15. MYCN amplification is detected by Taqman™ PCR using forward primer 5'-GTGCTCTCCAATTCTCGCCT-3', reverse primer 5'-GATGGCCTAGAGGAGGGCT-3' and probe 5'-FAM-CACTAAAGTTCCTTCCACCCTCTCCT-TAMRA-3' as described by Gotho *et al* (20).

For quantification of MYCN, any reference gene may be used. Preferably a reference gene located on chromosome 2 as the MYCN gene is used. Typically, the NAGK gene may be used as reference for quantification of MYCN by quantitative PCR methods. The GENBANK accession number for the NAGK gene is NM_017567. NAGK is for example detected by quantitative PCR using the forward and reverse primers of SEQ ID Nos. 16-17 and the quantitative PCR (TaqMan™) probe of SEQ ID No. 18. Typically, NAGK is quantified by Taqman™ PCR using forward primer 5'-TGGGCAGACACATCGTAGCA-3', reverse primer 5'-CACCTTCACTCCCACCTCAAC-3' and probe 5'-VIC-TGTTGCCCGAGATTGACCCGGT-TAMRA-3'.

The gain of genetic material from chromosome arm segment 17q21-qter may be determined by the quantification of any gene or of any sequence located on chromosome segment 17q21-qter.

In a first embodiment, the gain of genetic material from chromosome arm segment 17q21-qter is determined by quantification of the MPO gene located on 17q23.1 in comparison with a reference gene. The GENBANK accession number of the MPO gene is NG_009629. Preferably, the MPO gene is quantified by quantitative PCR using the forward and reverse primers of SEQ ID Nos. 1-2. The preferred quantitative PCR probe is preferably the probe of SEQ ID No. 3. Any appropriate method and any primer derived from the MPO gene may be used to quantify the MPO gene.

In another embodiment, the gain of genetic material from chromosome arm segment 17q21-qter is determined by quantification of the SURVIVIN gene located on 17q25 in comparison with a reference gene. The GENBANK accession number of the SURVIVIN gene is. U75285 Preferably, the SURVIVIN gene is quantified by quantitative PCR using the forward and reverse primers of SEQ ID Nos. 7-8. The preferred quantitative PCR probe is preferably the probe of SEQ ID No. 9. Any appropriate method and any primer derived from the SURVIVIN gene may be used to quantify the SURVIVIN gene.

Quantification of a gene from segment 17q21-qter is typically carried out in comparison with a reference gene. Any reference gene may be used in the methods of the present invention. Preferably, the reference gene is a gene for which no genetic aberrations or rearrangements have been described in neuroblastoma. Typically, the reference gene or the reference sequence is selected from a sequence or a gene on chromosome 17. Preferably, the gene or the sequence is on chromosome 17 but sufficiently distant from the 17q21-qter chromosomal segment.

In preferred embodiments, the reference gene is p53 located on 17p13.1. The GENBANK accession number of the p53 gene is U94788. Preferably, the p53 gene is quantified by quantitative PCR using the forward and reverse primers of SEQ ID Nos. 4-5. The preferred quantitative PCR probe is preferably the probe of SEQ ID No. 6. Any appropriate method and any primer derived from the p53 gene may be used to quantify the p53 gene.

Any gene quantification method may be used in the methods of the present invention. Preferably, a gain of genetic material from chromosome arm segment 17q21-qter is determined by quantitative amplification methods. After quantification/measurement of the target gene, such as MPO or SURVIVIN, and of the reference gene such as p53, the ratio between the amount of target sequence and the amount of reference sequence is calculated. A ratio above 1.2, 1.3, 1.4 or 1.5 is considered as indicative of a gain of genetic material from chromosome arm segment 17q21-qter.

Preferably, a gain of genetic material from chromosome arm segment 17q21-qter is determined by quantitative PCR using at least one primer selected from SEQ ID Nos. 1-2 and 7-8. Preferably, a gain of genetic material from chromosome arm segment 17q21-qter is determined by quantitative PCR using at least on probe selected from SEQ ID Nos. 3 and 9.

In the methods of the present invention, a gain of genetic material from chromosome arm segment 17q21-qter is associated with a poor prognosis. 17q gain is associated with a progressing neuroblastoma whereas a lack of 17q gain is associated with a stable or regressing neuroblastoma. Typically, in infants and toddlers under 18 months of age or under 2 years of age, the *"wait and see"* strategy may be recommended when both lack of MYCN amplification and lack of 17q gain are detected in circulating nucleic acids and if other factors are also favorable.

The present invention relates to a method for identifying a neuroblastoma and/or a neuroblastoma tumor having or prone to develop an invasive or metastatic phenotype.

The terms "invasive" or "aggressive" refer to a neuroblastoma or to a quickly growing tumor having a tendency to extend beyond its boundaries into adjacent tissues.

The term "metastatic" refers to the spread of a tumor from the organ of origin to additional organs/distal sites in the patient.

A "prognosis" is the likely course and outcome of a disease. The prognosis may include the likelihood of complications of the cancer, of metastasis, of spread, probable outcome of the cancer, likelihood of recovery, overall survival rate and /or overall death rate. Preferably, it is the probability that a patient will recover or have a recurrence/relapse of the cancer. This information is useful to the patient but also to the physician in determining the most effective course of treatment. A determination of the likelihood for a cancer relapse or of the likelihood of metastasis can assist the physician in determining whether a more conservative or a more radical approach to therapy should be taken. Prognosis provides for selection and classification of patients who are predicted to benefit from a given therapeutic regimen.

The methods of the present invention provide prognosis for neuroblastoma after it has been diagnosed and/or during therapeutic treatment.

A gain of genetic material from chromosome arm segment 17q21-qter is associated with reduced Progression Free Survival (PFS). When a gain of 17q is detected in the sample of circulating DNA, the probability of PFS is predicted as reduced in the human subject from whom the sample has been taken.

For example, human subjects having a gain of 17q as detected in their circulating nucleic acids have a 4 year Progression Free Survival rate of less than 70%, 50%, 45% or 40%. In these cases an aggressive therapeutic approach may be taken by the clinician.

A gain of genetic material from chromosome arm segment 17q21-qter is associated with reduced Overall Survival (OS). When a gain of 17q is detected in the sample of circulating DNA, the Overall Survival rate is predicted as reduced in the human subject from whom the sample has been taken.

For example, human subjects having a gain of 17q as detected in their circulating DNA have a 4 year Overall Survival rate of less than 90%, 80%, 75% or 70%. In these cases an aggressive therapeutic approach may be taken by the clinician.

A poor prognosis is that a neuroblastoma patient having a 17q gain has a higher probability to develop a cancer recurrence or an aggressive cancer than a patient having the same cancer without 17q gain. 17q gain detected in a sample of circulating DNA from the patient is correlated with a higher probability for relapse or recurrence of the cancer in the patient compared to a patient having the same cancer without 17q gain.

The present invention also relates to methods of determining the prognosis of a patient diagnosed with neuroblastoma or suspected of suffering from neuroblastoma comprising:
a) Obtaining a sample of circulating DNA from said patient,
b) Determining whether there is a gain of genetic material from chromosome segment 17q-qter in said sample,
c) Correlating the gain of genetic material from chromosome segment 17q-qter with a poor prognosis.

The present invention also relates to methods of estimating the Progression Free Survival rate (PFS) of a patient diagnosed with neuroblastoma or suspected of suffering from neuroblastoma comprising:
a) Obtaining a sample of circulating DNA from said patient,
b) Determining whether there is a gain of genetic material from chromosome segment 17q-qter in said sample,
c) Correlating the gain of genetic material from chromosome segment 17q-qter with a reduced PFS.

The PFS in a patient diagnosed with a 17q gain is reduced compared to a patient who has not a 17q gain.

The present invention also relates to methods of estimating the Overall Survival rate (OS) of a patient diagnosed with neuroblastoma or suspected of suffering from neuroblastoma comprising:
a) Obtaining a sample of circulating DNA from said patient,
b) Determining whether there is a gain of genetic material from chromosome segment 17q-qter in said sample,
c) Correlating the gain of genetic material from chromosome segment 17q-qter with a reduced OS.

The OS in a patient diagnosed with a 17q gain is reduced compared to a patient who has not a 17q gain.

In preferred embodiments the methods of the present invention are performed after MYCN amplification testing and 17q gain testing is performed solely for patients suffering from non-MYCN amplified neuroblastoma.

### EXAMPLES

### 1) Patients and methods

### Patients

Patients diagnosed with NB were enrolled in the study with the informed consent of their parents. This study was conducted under research protocols approved by each institutional review board. Plasma from 16 volunteer controls and serum or plasma from 143 patients with neuroblastoma, for which the genomic classification of tumors is known, were assayed. All samples (serum, plasma and tumors) were obtained at diagnosis except for one patient where they were obtained at relapse.

Serum or plasma samples were prepared by centrifugation of blood at 2000rpm for 10 min at 4°C followed by careful aliquoting and freezing at -80°C.

### DNA isolation

Tumoral DNA was extracted from tissues samples using phenol chloroform extraction and circulating DNA was extracted from 200µl of plasma or serum using the QIAmp blood kit (Qiagen, Courtaboeuf, France) according to the blood and body fluid protocol recommended by the manufacturer.

### PCR amplification

Circulating DNA was analyzed by duplex quantitative real-time PCR. The genes MPO and Survivin located on 17q23.1 and 17q25 respectively were defined as target genes since the region of gain extends from 17q23.1 to 17 qter was described for promoting tumor progression. The gene p53 was chosen as reference gene because it is on the same chromosome (17p13.1 than the target genes but sufficiently distant from the region with a gain. Moreover no aberration of the p53 gene in neuroblastoma has ever been found, and the gene status in neuroblastoma is known to be stable. Specific oligonucleotide primers and probes previously described by Morowitz *et al.* and Tajiri *et al.* are shown in table 1.

**Table 1 : Q-PCR oligonucleotide Sequences and Fluorescence Dyes**

| | |
|---|---|
| ***MPO* locus at 17q23.1** | |
| | |
| Forward Primer | AGGGCTCCTGGCCATTCT |
| Reverse Primer | AACCACCCCAACACACCATCT |
| Q-PCR Probe | *FAM*-AGGTGCTGCTCCAGGTAACAGTTCCCA-*TAMRA* |
| | |
| ***TP53* locus at 17p13.1** | |
| | |
| Forward Primer | TGTCCTTCCTGGAGCGATCT |
| Reverse Primer | CAAACCCCTGGTTTAGCACTTC |
| Q-PCR Probe | *Yakima yellow*-CAGCCCCCGGCTCCGCTAGA-*TAMRA* |
| | |
| **Survivin gene at 17q25** | |
| | |
| Forward Primer | 5'-GGGCTGCCACGTCCAC-3' |
| Reverse Primer | 5'-GTCGTCATCTGGCTCCCA-3' |
| Q-PCR Probe | 5'-FAM-TTCATCCACTGCCCCACTGAGAACGA-*TAMRA-*3' |

TaqMan PCR was performed using the ABI Prism 7000 Sequence Detection System (Applied Biosystems, Foster City, CA). Amplification mixtures (25 µl) consisted of template DNA (5 µl), 1x TaqMan Universal PCR MasterMix (Applied Biosystems), 360 nmol/L of each primer, and 100 nmol/L of fluorogenic probe. All PCR reactions were performed with one cycle of 95°C for 10 minutes, followed by PCR amplification with 40 cycles of 95°C for 15 seconds and 60°C for 1 minute. Standard curves were constructed in each PCR run with four-fold serial dilutions containing 100, 10, 1 and 0.1 ng/µL of a healthy donor's DNA. The dosages of the genes in each sample were extrapolated from these standard curves. Two measurements for each gene were performed and an average was calculated before to define the ratio (target/reference). A ratio superior to 1.35 was considered indicative of a high level of circulating MPO or survivin DNA sequences and a surrogate for tumoral 17q gain.

### CGH analysis

Tumor cell content was greater than 60% in all analyzed samples all tumors were investigated on the array-CGH platform of the Curie Institute with at least 2,855 BACs, corresponding to a genomic resolution of approximately 1 megabase.

### MLPA analysis

For this multigenomic technique, a specifically designed set of 36 probes was used to test chromosomal abnormalities on chromosomes 2 and 17 (SALSA MLPA Kit/P252 (MRCHolland Amsterdam, The Netherlands). MLPA was performed as described by the manufacturer. Briefly, 4 steps (denaturation, hybridization, ligation and amplification) were performed before the separation of final PCR fragments by capillary electrophoresis on an ABI-Prism 3130 Genetic Analyzer (Applied Biosystems). Peak area and height were measured using Genemapper analysis software (Applied Biosystems). Data were analyzed with MLPA Vizard Software (Austrian Research Centers, Vienna) using control DNA from five healthy donors, and relative copy number (RCN) values were calculated.

### 2) Results

The accuracy of our study was evaluated by analysis of plasma from 16 volunteer controls. The ratio detected for MPO were 0.36 to 1.00 and the ratio detected for Survivin were 0.48 to 1.14. Using a cut-off of 1.35 for defining the samples with 17q gain, 43 positive cases have been detected among the 143 serum/plasma samples tested. When we have compared the status of chromosome 17 obtained by aCGH or MLPA on the tumors and the one obtained by duplex quantitative real-time PCR on circulating DNA, we observed that the 17q gain was detected in 31 of 59 serum/plasma samples from patients with 17q gain on tumors, 12 of 84 samples from neuroblastoma patients without 17q gain. Thirty-three serum/plasma samples were obtained from patients with stages 1 and 2, 29 from patients with INSS stage 3 disease, 62 from patients with INSS stage 4 and 10 from patients with INSS stage 4s. For 9 patients the stage was unknown. As shown in table 2A, the sensitivity increased with the stage of the disease. It varied from 33% to 58% whereas the specificity was from 71% to 100%. A perfect specificity was observed in stage 3 and 4s. We have evaluated the specificity and sensitivity in the children populations under and over 18 months. As shown in table 2B, in patients less than 18 months of age serum based determination of 17q gain had good specificity (93 %) and sensitivity of 55% whereas for the population more than 18 months of age, the specificity and sensitivity were respectively of 69% and 51 %

Determination of the 17q gain in the peripheral blood could facilitate the prediction of prognosis in particular in patients without MYCN amplification neuroblastoma. Among the 143 patients enrolled in the study, 121 had non-MNA tumors and 41 samples had a 17q gain on the tumors detected by a CGH or MLPA. Similar sensitivity and specificity were observed between the populations with or without MYCN amplification. In the children populations under and over 18 months, a high specificity (94.7%) and sensitivity of 53.8% were observed in infants and toddlers (p≤0.001). For children more than 18 months of age, a poor sensitivity and a poor specificity were detected (p=ns) (table 2C).

**Table 2. Detection of a 17q gain in the peripheral blood of neuroblastoma patients. 2A) according to the stage of the disease, 2B) according to the age of the patients at diagnosis, 2C) in neuroblastoma patients without MYCN amplification and according to the age of the patients at diagnosis.**

| **2A** | **17q normal/17q gain tumor** | **17q gain tumor** |
|---|---|---|
| | **n = 84** | **n =59** |
| **Stages 1 and 2 (*n*=33)** | **27** | **6** |
| - No detection of 17q gain in circulating DNA | 24 | 4 |
| - Detection of 17q gain in circulating DNA | 3 | 2 |
| **p=NS** | Specificity = 88% | Sensitivity = 33% |
| **Stage 3 (*n*=29)** | **19** | **10** |
| - No detection of 17q gain in circulating DNA | 19 | 6 |
| - Detection of 17q gain in circulating DNA | 0 | 4 |
| **p≤0.01** | Specificity = 100% | Sensitivity = 40% |
| ***Stage 4 (n=62)*** | **21** | **41** |
| - No detection of 17q gain in circulating DNA | 15 | 17 |
| - Detection of 17q gain in circulating DNA | 6 | 24 |
| **p≤0.05** | Specificity = 71.4% | Sensitivity = 58.5% |
| ***Stage 4 S (n=10)*** | **8** | **2** |
| - No detection of 17q gain in circulating DNA | 8 | 1 |
| - Detection of 17q gain in circulating DNA | 0 | 1 |
| **p≤0.05** | Specificity = 100% | Sensitivity = 50% |
| ***Stage unknown (n =9)*** | **9** | **0** |
| - No detection of 17q gain in circulating DNA | 6 | 0 |
| - Detection of 17q gain in circulating DNA | 3 | 0 |
| | Specificity = 66% | Sensitivity = NE |
| **p= NE** | | |

| **2B** | **17q normal/17q gain tumor** | **17q gain tumor** |
|---|---|---|
| | **n=84** | **n=59** |
| **≤18 months (*n*=76)** | **58** | **18** |
| - No detection of 17q gain in circulating DNA | 54 | 8 |
| - Detection of 17q gain in circulating DNA | 4 | 10 |
| **p≤0.001** | Specificity = 93% | Sensitivity = 55% |
| **>18 months (*n*=67)** | **26** | **41** |
| - No detection of 17q gain in circulating DNA | 18 | 20 |
| - Detection of 17q gain in circulating DNA | 8 | 21 |
| **p=NS** | Specificity = 69% | Sensitivity = 51% |
| | | |

| **2C** | **17q normal/17q gain tumor** | **17q gain tumor** |
|---|---|---|
| | **n =80** | **n =41** |
| **≤18 months (*n*=70)** | **57** | **13** |
| - No detection of 17q gain in circulating DNA | 54 | 6 |
| - Detection of 17q gain in circulating DNA | 3 | 7 |
| **p≤0.001** | Specificity = 94.7% | Sensitivity = 53.8% |
| **>18 months (*n*=51)** | **23** | **28** |
| - No detection of 17q gain in circulating DNA | 16 | 14 |
| - Detection of 17q gain in circulating DNA | 7 | 14 |
| **p=NS** | Specificity = 69.5% | Sensitivity = 50% |

### REFERENCES

### Non-patent references

1. Schleiermacher G, Michon J, Huon I, et al. Chromosomal CGH identifies patients with a higher risk of relapse in neuroblastoma without MYCN amplification. Br J Cancer. 2007, 97:238-46
2. Mosse YP, Diskin SJ, Wasserman N, et al Neuroblastomas have distinct genomic DNA profiles that predict clinical phenotype and regional gene expression. Genes Chromosomes Cancer. 2007,46:936-49.
3. Tomioka N, Oba S, Ohira M, et al. Novel risk stratification of patients with neuroblastoma by genomic signature,which is independent of molecular signature.Oncogene. 2008 ;27:441-9.
4. Janoueix-Lerosey I, Schleiermacher G, Michels E, Mosseri V, Ribeiro A, Lequin D, Vermeulen J, Couturier J, Peuchmaur M, Valent A, Plantaz D, Rubie H, Valteau-Couanet D, Thomas C, Combaret V, Rousseau R, Eggert A, Michon J, Speleman F, Delattre O. Overall genomic pattern is a predictor of outcome in neuroblastoma. J Clin Oncol. 2009 Mar 1;27(7):1026-33.
5. Bown N, Cotterill S, Lastowska M, O'Neill S, Pearson AD, Plantaz D, Meddeb M, Danglot G, Brinkschmidt C, Christiansen H, Laureys G, Speleman F. 1999. Gain of chromosome arm 17q and adverse outcome in patients with neuroblastoma. N Engl J Med 340: 1954-1961
6. Brinkschmidt C, Christiansen H, Terpe, HJ, Simon R, Boecker W, Lampert F, Stoerkel S. 1997. Comparative genomic hybridization (CGH) analysis of neuroblastomas - an important methodological approach in paediatric tumour pathology. J Pathol 181: 394-400
7. Lastowska M, Roberts P, Pearson AD, Lewis I, Wolstenholme J, Bown N. 1997. Promiscuous translocations of chromosome arm 17q in human neuroblastomas. Genes Chromosomes Cancer- 19: 143-149.
8. Trakhtenbrot L, Cohen N, Betts DR, Niggli FK, Amariglio N, Brok-Simoni F, Rechavi G, Meitar D. 2002. Interphase fluorescence in situ hybridization detection of chromosome 17 and 17q region gains in neuroblastoma: are they secondary events? Cancer Genet Cytogenet 137: 95-101
9. Lastowska M, Cullinane C, Varind S, Cotterill S, Bown N, O'Neill S, Mazzocco K, Roberts P, Nicholson J, Ellershaw C, Pearson ADJ, Jackson M. 2001. Comprehensive genetic and histopathologic study reveals three types of neuroblastoma tumors. J Clin Oncol 19: 3080-3090.
10. Leon, S. A., Shapiro, B., Sklaroff, D. M., and Yaros, M. J. Free DNA in the serum in cancer patients and the effect of therapy. Cancer Res., 37: 646-650, 1977.
11. Chen, X. Q., Stroun, M., Magnenat, J. L., Nicod, L. P., Kurt, A. M., Lyautey, J., Lederrey, C., and Anker, P. Microsatellite alterations in plasma DNA of small cell lung cancer patients. Nat. Med., 2: 1033-1035, 1996.
12. Silva, J. M., Dominguez, G., Garcia, J.M., Gonzalez, R., Villanueva, M. J., Navarro, F., Provencio, M., San Martin, S., Espana, P., and Bonilla, F. Presence of tumor DNA in plasma of breast cancer patients: clinicopathological correlations. Cancer Res., 59: 3251-3256, 1999.
13. Chen, X., Bonnefoi, H., Diebold-Berger, S., Lyautey, J., Lederrey, C., Faltin-Traub, E., Stroun, M., and Anker P. Detecting tumor-related alterations in plasma or serum DNA of patients diagnosed with breast cancer. Clin. Cancer Res., 5: 2297-2303, 1999.
14. Nawroz, H., Koch, W., Anker, P., Stroun, M., and Sidransky, D. Microsatellite alterations in serum DNA of head and neck cancer patients. Nat. Med., 2: 1035-1037, 1996.
15. Fujiwara, Y., Chi, D. D. J., Wang, H., Keleman, P., Morton, D. L., Turner, M. R., and Hoon, D. S. Plasma DNA microsatellites as tumor-specific markers and indicators of tumor progression in melanoma patients, Cancer Res., 59: 1567-1571, 1999.
16. Sozzi, G., Musso, K., Ratcliffe, C., Goldstraw, P., Pierotti, A., Pastorino, U. Detection of microsatellite alterations in plasma DNA of non-small cell lung cancer patients : a prospect for early diagnosis, Clin. Cancer Res., 5: 2689-2692, 1999.
17. Coulet, F., Blons, H., Cabelguenne, A., Lecomte, T., Laccourreye, O., Brasnu, D., Beaune, P., Zucman, J., and Laurent-Puig, P. Detection of plasma tumor DNA in head and neck squamous cell carcinoma by microsatellite typing and p53 mutation analysis, Cancer Res., 60: 707-711, 2000.
18. Combaret V, Bergeron C, Noguera R,et al. Circulating MYCN DNA as a tumor-specific marker in neuroblastoma patients. Cancer Res 2002;62:3646-3648.
19. Combaret V, Audoynaud C, Iacono I, et al. Circulating MYCN DNA predicts MYCN-amplification in neuroblastoma. J Clin Oncol 2005;23:8919-8920.
20. Gotoh T, Hosoi H, Iehara T, et al. Prediction of MYCN amplification in neuroblastoma using serum DNA and real-time quantitative polymerase chain reaction. J Clin Oncol 2005;23:5205-5210.
21. Combaret V, Hogarty MD, London WB, McGrady P, Iacono I, Brejon S, Swerts K, Noguera R, Gross N, Rousseau R, Puisieux A. Influence of neuroblastoma stage on serum-based detection of MYCN amplification. Pediatr Blood Cancer. 2009 Sep;53(3):329-31.

## Claims

1. Method for *in vitro* diagnosis of neuroblastoma comprising determining whether there is a gain of genetic material from chromosome segment 17q21-qter in a sample of circulating nucleic acids from a human subject under 18 months of age wherein a gain of genetic material from chromosome arm segment 17q21-qter is associated with a poor prognosis.

2. Method for *in vitro* diagnosis of neuroblastoma according to claim 1 wherein the sample of circulating nucleic acids is a sample of circulating DNA.

3. Method for *in vitro* diagnosis of neuroblastoma according to anyone of claims 1-2 wherein the sample of circulating nucleic acids is negative for MYCN gene amplification.

4. Method for *in vitro* diagnosis of neuroblastoma according to anyone of claims 1-3 wherein the gain of genetic material from chromosome segment 17q21-qter is determined by quantification of the MPO gene located on 17q23.1 in comparison with a reference gene.

5. Method for *in vitro* diagnosis of neuroblastoma according to anyone of claims 1-4 wherein the gain of genetic material from chromosome arm segment 17q21-qter is determined by quantification of the SURVIVIN gene located on 17q25 in comparison with a reference gene.

6. Method for *in vitro* diagnosis of neuroblastoma according to anyone of claims 4-5 wherein the reference gene is selected from a gene on chromosome 17.

7. Method for *in vitro* diagnosis of neuroblastoma according to anyone of claims 4-6 wherein the reference gene is p53 located on 17p13.1.

8. Method for *in vitro* diagnosis of neuroblastoma according to anyone of claims 1-7 wherein a gain of genetic material from chromosome segment 17q21-qter is determined by quantitative amplification methods.

9. Method for *in vitro* diagnosis of neuroblastoma according to anyone of claims 1-8 wherein a gain of genetic material from chromosome arm segment 17q21-qter is determined by quantitative PCR using primers of SEQ ID Nos. 1-2 and probe of SEQ ID no. 3.

10. Method for *in vitro* diagnosis of neuroblastoma according to anyone of claims 1-9 wherein a gain of genetic material from chromosome arm segment 17q21-qter is determined by quantitative PCR using primers of SEQ ID Nos. 7-8 and probe of SEQ ID no. 9.

11. Method for *in vitro* diagnosis of neuroblastoma according to anyone of claims 1-10 wherein a gain of genetic material from chromosome arm segment 17q21-qter is associated with reduced Progression Free Survival (PFS).

12. Method for *in vitro* diagnosis of neuroblastoma according to anyone of claims 1-10 wherein a gain of genetic material from chromosome arm segment 17q21-qter is associated with reduced Overall Survival (OS).

## Patentansprüche

1. Verfahren zur Diagnose eines Neuroblastoms *in vitro,* umfassend die Bestimmung, ob in einer Probe zirkulierender Nukleinsäuren von einem humanen Subjekt im Alter von unter 18 Monaten ein Zuwachs an genetischem Material von dem Chromosomensegment 17q21-qter vorliegt, wobei ein Zuwachs an genetischem Material von dem Chromosomenarmsegment 17q21-qter mit einer schlechten Prognose assoziiert wird.

2. Verfahren zur Diagnose eines Neuroblastoms *in vitro* nach Anspruch 1, wobei die Probe zirkulierender Nukleinsäuren eine Probe zirkulierender DNA ist.

3. Verfahren zur Diagnose eines Neuroblastoms *in vitro* nach einem der Ansprüche 1 bis 2, wobei die Probe zirkulierender Nukleinsäuren für die MYCN-Genamplifikation negativ ist.

4. Verfahren zur Diagnose eines Neuroblastoms *in vitro* nach einem der Ansprüche 1 bis 3, wobei der Zuwachs an genetischem Material von dem Chromosomensegment 17q21-qter durch Quantifizierung des auf 17q23.1 lokalisierten MPO-Gens im Vergleich mit einem Referenzgen bestimmt wird.

5. Verfahren zur Diagnose eines Neuroblastoms *in vitro* nach einem der Ansprüche 1 bis 4, wobei der Zuwachs an genetischem Material von dem Chromosomenarmsegment 17q21-qter durch Quantifizierung des auf 17q25 lokalisierten SURVIVIN-Gens im Vergleich mit einem Referenzgen bestimmt wird.

6. Verfahren zur Diagnose eines Neuroblastoms *in vitro* nach einem der Ansprüche 4 bis 5, wobei das Referenzgen aus einem Gen aus Chromosom 17 ausgewählt ist.

7. Verfahren zur Diagnose eines Neuroblastoms *in vitro* nach einem der Ansprüche 4 bis 6, wobei das Referenzgen das auf 17p13.1 lokalisierte p53 ist.

8. Verfahren zur Diagnose eines Neuroblastoms *in vitro* nach einem der Ansprüche 1 bis 7, wobei ein Zuwachs an genetischem Material von dem Chromosomensegment 17q21-qter durch quantitative Amplifikationsverfahren bestimmt wird.

9. Verfahren zur Diagnose eines Neuroblastoms *in vitro* nach einem der Ansprüche 1 bis 8, wobei ein Zuwachs an genetischem Material von dem Chromosomenarmsegment 17qZ1-qter durch quantitative PCR unter Verwendung der Primer von SEQ ID Nr. 1-2 und der Sonde von SEQ ID Nr. 3 bestimmt wird.

10. Verfahren zur Diagnose eines Neuroblastoms *in vitro* nach einem der Ansprüche 1 bis 9, wobei ein Zuwachs an genetischem Material von dem Chromosomenarmsegment 17q21-qter durch quantitative PCR unter Verwendung der Primer von SEQ ID Nr. 7-8 und der Sonde von SEQ ID Nr. 9 bestimmt wird.

11. Verfahren zur Diagnose eines Neuroblastoms *in vitro* nach einem der Ansprüche 1 bis 10, wobei ein Zuwachs an genetischem Material von dem Chromosomenarmsegment 17q21-qter mit Reduziertem Progressionsfreiem Überleben (PFS) assoziiert wird.

12. Verfahren zur Diagnose eines Neuroblastoms *in vitro* nach einem der Ansprüche 1 bis 10, wobei ein Zuwachs an genetischem Material von dem Chromosomenarmsegment 17q21-qter mit Reduziertem Gesamtüberleben (OS) assoziiert wird.

## Revendications

1. Procédé de diagnostic *in vitro* de neuroblastome, comprenant la détermination du fait qu'il existe ou non un gain de matériel génétique issu du segment de chromosome 17q21-qter dans un échantillon d'acides nucléiques circulants d'un sujet humain de moins de 18 mois, dans lequel un gain de matériel génétique issu du segment de bras de chromosome 17q21-qter est associé à un pronostic défavorable.

2. Procédé de diagnostic *in vitro* de neuroblastome selon la revendication 1, dans lequel l'échantillon d'acides nucléiques circulants est un échantillon d'ADN circulant.

3. Procédé de diagnostic *in vitro* de neuroblastome selon l'une quelconque des revendications 1 à 2, dans lequel l'échantillon d'acides nucléiques circulants est négatif pour l'amplification du gène MYCN.

4. Procédé de diagnostic *in vitro* de neuroblastome selon l'une quelconque des revendications 1 à 3, dans lequel le gain de matériel génétique issu du segment de chromosome 17q21-qter est déterminé par quantification du gène MPO situé sur 17q23.1 par rapport à un gène de référence.

5. Procédé de diagnostic *in vitro* de neuroblastome selon l'une quelconque des revendications 1 à 4, dans lequel le gain de matériel génétique issu du segment du bras de chromosome 17q21-qter est déterminé par quantification du gène SURVIVIN situé sur 17q25 par rapport à un gène de référence.

6. Procédé de diagnostic *in vitro* de neuroblastome selon l'une quelconque des revendications 4 à 5 dans lequel le gène de référence *est choisi* à partir d'un gène sur le chromosome 17.

7. Procédé de diagnostic *in vitro* de neuroblastome selon l'une quelconque des revendications 4 à 6 dans lequel le gène de référence est p53 situé sur 17p13.1.

8. Procédé de diagnostic *in vitro* de neuroblastome selon l'une quelconque des revendications 1 à 7, dans lequel un gain de matériel génétique issu du segment 17q21-qter de chromosome est déterminé par des procédés d'amplification quantitatifs.

9. Procédé de diagnostic *in vitro* de neuroblastome selon l'une quelconque des revendications 1 à 8, dans lequel un gain de matériel génétique issu du segment du bras de chromosome 17q21-qter est déterminé par PCR quantitative en utilisant des amorces de SEQ ID N° : 1 et 2 et une sonde de SEQ ID N° 3.

10. Procédé de diagnostic *in vitro* de neuroblastome selon l'une quelconque des revendications 1 à 9, dans lequel un gain de matériel génétique issu du segment du bras de chromosome 17q21-qter est déterminé par PCR quantitative en utilisant des amorces de SEQ ID N° : 7 et 8 et une sonde de SEQ ID N° 9.

11. Procédé de diagnostic in vitro de neuroblastome selon l'une quelconque des revendications 1 à 10, dans lequel un gain de matériel génétique issu du segment du bras de chromosome 17q21-qter est associé à une réduction de la survie sans progression (SSP).

12. Procédé de diagnostic *in vitro* de neuroblastome selon l'une quelconque des revendications 1 à 10, dans lequel un gain de matériel génétique issu du segment du bras de chromosome 17q21-qter est associé à une réduction de la survie globale (SG).
